# EUROPEAN PATENT APPLICATION

(11) **EP 4 591 784 A2**
(43) Date of publication of application: **30.07.2025**
(21) Application number: 25182966.9
(22) Date of filing: 01.09.2022
(51) Int. Cl.: A61B 5/00

(54) **SYSTEMS AND METHODS FOR SENSING DEFECATION EVENTS**

(30) Priority: 03.09.2021 GR 20210100581; 14.09.2021 US 202163261154 P
(62) Divisional of application: 22778121.8
(71) Applicant: Eli Lilly and Company, Indianapolis, IN 46285 (US)
(72) Inventor: ANNETTA, Nicholas Vincent, Indianapolis, 46206-6288 (US); ASH, Matthew James, Indianapolis, 46206-6288 (US); BAKER, Cory William, Indianapolis, 46206-6288 (US); BARUCH, Uri Eliezer, Indianapolis, 46206-6288 (US); BRANCH, Joshua Richard, Indianapolis, 46206-6288 (US); DANG, Xiangnan, Indianapolis, 46206-6288 (US); DAVIS, Yelena Nikolayevna, Indianapolis, 46206-6288 (US); FERNANDEZ-MARTOS BALSON, Maria, Indianapolis, 46206-6288 (US); FORDHAM, Matthew Keith, Indianapolis, 46206-6288 / (US); FORTNEY, Clark Edward, Indianapolis, 46206-6288 (US); FUNKHOUSER, Chloe Marie, Indianapolis, 46206-6288 (US); GOTTLIEB, Klaus Theodor, Indianapolis, 46206-6288 (US); HART, Alison Claire, Indianapolis, 46206-6288 (US); HUCKABY, Steven Eldridge, Indianapolis, 46206-6288 (US); KOURTIS, Iraklis, Indianapolis, 46206-6288 (US); KOURTIS, Lampros, Indianapolis, 46206-6288 (US); KUTE, Stephanie Michelle, Indianapolis, 46206-6288 (US); LANHAM, Christopher Shane, Indianapolis, 46206-6288 (US); MEYERS, Eric Christopher, Indianapolis, 46206-6288 (US); OWEN, Philip James, Indianapolis, 46206-6288 (US); PLATFOOT, Nathan Joseph, Indianapolis, 46206-6288 (US); PLATT, Jessica Alice, Indianapolis, 46206-6288 (US); SHELFORD, Leigh Robert, Indianapolis, 46206-6288 (US); SPURBECK, Rachel Rebecca, Indianapolis, 46206-6288 (US); STEARN, Thomas Jack, Indianapolis, 46206-6288 (US); WINGER, Brian Ellis, Indianapolis, 46206-6288 (US); YANG, Jian, Indianapolis, 46206-6288 (US)
(74) Representative: J A Kemp LLP

(57) **Abstract**

System for sensing defecation events of a subject are disclosed. In one arrangement, a system comprises: a wearable device configured to be carried on a torso of the subject and under lower-body clothing worn by the subject, the wearable device comprising an optical sensor configured to sense increased light when the subject removes the lower-body clothing; and a processor operably coupled to the optical sensor, the processor configured to determine occurrence of defecation events based at least in part on the increased light sensed by the optical sensor.

## Description

### FIELD OF THE DISCLOSURE

The present disclosure relates to systems and methods for automatically sensing defecation events of subjects. More particularly, the present disclosure relates to systems and methods for sensing defecation events by sensing one or more actions associated with such events.

### BACKGROUND OF THE DISCLOSURE

Accurately obtaining defecation event data (for example, the frequency and/or timing of such events) is typically required for diagnosis, assessment, treatment and/or management for various digestive diseases, such as irritable bowel syndrome (IBS), inflammatory bowel disease (IBD), and chronic constipation. However, obtaining such data can be cumbersome and typically relies on patient recall, which can lead to inaccuracy.

### SUMMARY OF THE DISCLOSURE

The present disclosure provides systems and methods for easily and accurately sensing defecation events and obtaining defecation event data. These systems and methods need not rely on patient recall.

According to an embodiment of the present disclosure, a system for sensing defecation events of a subject includes a wearable device configured to be carried on the torso of the subject. The wearable device is operable in a sleep mode and an active mode. The wearable device includes a wake-up sensor that is configured to sense a first stimulus and a mechanomyogram (MMG) sensor configured to sense abdominal muscle movement signals of the subject. A processor is operably coupled to the wake-up sensor and the MMG sensor. The processor reconfigures the wearable device from the sleep mode to the active mode based on the first stimulus sensed by the wake-up sensor. In the active mode the wearable device is configured to communicate with the processor to determine occurrence of defecation events of the subject based on abdominal muscle movement signals sensed by the MMG sensor.

In some embodiments, the abdominal muscle movement signals of the subject is a second stimulus, and the system further includes a third sensor operably coupled to the processor and configured to sense a third stimulus. In the active mode the processor is configured to determine occurrence of defecation events of the subject based on abdominal muscle movement signals sensed by the mechanomyogram sensor and the third stimulus sensed by the third sensor.

In some embodiments, the third sensor comprises a gas sensor disposed in the wearable device and configured to sense flatus.

In some embodiments, the third sensor is an audio sensor configured to sense toilet flushing sounds. In some embodiments, the audio sensor may also be configured to sense sounds caused by or associated with movement of clothing, e.g., the rustling of lower body clothing as it is removed.

In some embodiments, the third sensor is an electromyogram electrode configured to sense muscle electrical signals of the subject.

In some embodiments, the third sensor is an inertial measurement unit configured to sense a change in posture of the subject.

In some embodiments, the wearable device further includes a patch configured to be carried on the torso of the subject, and the patch carries the wake-up sensor and the mechanomyogram sensor.

In some embodiments, the patch further carries the processor.

In some embodiments, the wearable device further includes a belt configured to extend around the torso of the subject, and the belt carries the wake-up sensor and the mechanomyogram sensor.

In some embodiments, the belt further carries the processor.

In some embodiments, the wake-up sensor includes one of an optical sensor and a resistive force sensor configured to sense when the subject removes lower-body clothing.

In some embodiments, the wearable device further includes a health sensor configured to sense a health stimulus associated with health of the subject.

In some embodiments, the health sensor includes a blood sensor configured to sense blood in stool of the subject.

In some embodiments, the blood sensor includes a solid-state vapor detection sensor configured to sense one or more volatile organic compounds.

According to another embodiment of the present disclosure, a system for sensing defecation events of a subject includes a wearable device configured to be carried on the torso of the subject. The wearable device includes a mechanomyogram sensor configured to sense abdominal muscle movement signals of the subject and a gas sensor configured to sense flatus. A processor is operably coupled to the mechanomyogram sensor and the gas sensor. The processor is configured to determine occurrence of defecation events of the subject based on abdominal muscle movement signals sensed by the mechanomyogram sensor and flatus sensed by the gas sensor.

In some embodiments, the processor is configured to determine occurrence of defecation events of the subject based on a sequence of events comprising one of the abdominal muscle movement signals sensed by the mechanomyogram sensor and the flatus sensed by the gas sensor preceding the other of the abdominal muscle movement signals sensed by the mechanomyogram sensor and the flatus sensed by the gas sensor.

In some embodiments, the wearable device further includes a base carrying the mechanomyogram sensor, the gas sensor, and the processor.

According to yet another embodiment of the present disclosure, a system for sensing defecation events of a subject includes a wearable device configured to be carried on the torso of the subject and under lower-body clothing worn by the subject. The wearable device includes an optical sensor configured to sense increased light when the subject removes the lower-body clothing. A processor is operably coupled to the optical sensor, and the processor is configured to determine occurrence of defecation events based at least in part on the increased light sensed by the optical sensor.

In some embodiments, the wearable device is operable in an active mode and a sleep mode, and the processor reconfigures the wearable device from the sleep mode to the active mode upon determining removal of the lower-body clothing by the subject in response to the increased light sensed by the optical sensor.

In some embodiments, the optical sensor is a first sensor configured to sense light as a first stimulus. The wearable device further includes a second sensor configured to sense a second stimulus when the wearable device is in the active mode. The second stimulus is different than the first stimulus. The processor is operably coupled to the second sensor, and the processor is configured to determine occurrence of defecation events of the subject based on signals received from the second sensor.

In some embodiments, the optical sensor is a first sensor configured to sense light as a first stimulus. The wearable device further includes a second sensor configured to sense a second stimulus when the wearable device is in the active mode. The second stimulus is different than the first stimulus. The processor is operably coupled to the second sensor, and the processor is configured to determine occurrence of defecation events of the subject based on signals received from the first sensor and the second sensor.

In some embodiments, the second sensor is a mechanomyogram sensor.

In some embodiments, the wearable device further includes a patch configured to be carried on the torso of the subject, and the patch carries the optical sensor and the processor.

In some embodiments, the wearable device further comprises a belt configured to extend around the torso of the subject, the belt carrying the optical sensor and the processor.

According to yet another embodiment of the present disclosure, a method for sensing a defecation event of a subject includes: sensing, by an optical sensor of a wearable device carried on the torso of the subject, increased light when the subject removes lower-body clothing; sensing, by a mechanomyogram sensor of the wearable device, abdominal muscle movement signals of the subject; and determining occurrence of the defecation event, at least in part, based on the sensed increased light when the subject removes the lower-body clothing and the sensed abdominal muscle movement signals of the subject.

In some embodiments, the method further includes sensing, by a gas sensor of the wearable device, flatus, and the determination that the defecation event has occurred is based at least in part on the sensed flatus.

In some embodiments, the method further includes sensing, by an inertial measurement unit of the wearable device, a sitting motion by the subject before sensing the abdominal muscle movement signals of the subject, and the determination that the defecation event has occurred is based at least in part on the sensed sitting motion.

In some embodiments, the method further includes sensing, by an inertial measurement unit of the wearable device, a standing motion by the subject after sensing the flatus, and the determination that the defecation event has occurred is based at least in part on the sensed standing motion.

In some embodiments, the method further includes sensing a plurality of defecation events of the subject over a time period.

In some embodiments, sensing each of the plurality of defecation events of the subject comprises: sensing, by the mechanomyogram sensor of the wearable device, abdominal muscle movement signals of the subject; sensing, by the optical sensor, increased light when the subject removes lower-body clothing; and determining occurrence of each of the plurality of defecation events, at least in part, based on the sensed abdominal muscle movement signals of the subject and the sensed increased light when the subject removes the lower-body clothing.

In some embodiments, sensing, by the optical sensor, the increased light when the subject removes the lower-body clothing precedes sensing, by the mechanomyogram sensor, the abdominal muscle movement signals of the subject.

In some embodiments, sensing, by the mechanomyogram sensor, the abdominal muscle movement signals of the subject precedes sensing, by the optical sensor, the increased light when the subject removes the lower-body clothing.

In some embodiments, the method further includes reconfiguring the wearable device from a sleep mode to an active mode based on the sensed increased light when the subject removes the lower-body clothing, in the sleep mode the mechanomyogram sensor being inactive, and in the active mode the mechanomyogram sensor being configured to sense the abdominal muscle movement signals of the subject.

According to yet another embodiment of the present disclosure, a system for training one or more processors to detect defecation events of a subject includes a first wearable device configured to be carried on the body of the subject. The first wearable device includes a first defecation event sensor configured to sense one or more first stimuli. The system further includes a second wearable device configured to be carried on the body of the subject. The second wearable device includes a second defecation event sensor configured to sense one or more second stimuli. The system further comprises one or more processors operably coupled to the first defecation event sensor and the second defecation event sensor and configured to determine occurrence of a detected defecation event of the subject based on the one or more second stimuli, and to associate first stimuli sensed by the first defecation event sensor within a predetermined time period of the detected defecation event with defecation events of the subject.

In some embodiments, the one or more processors are further configured to train a machine-learning algorithm for detecting defecation events of the subject with data indicative of first stimuli sensed by the first defecation event sensor that has been associated with defecation events of the subject.

In some embodiments, the first wearable device further includes a wake-up sensor configured to sense one or more wake-up stimuli. The first wearable device is configured to transition from a sleep mode to an active mode in which the first wearable device is configured to sense defecation events of the subject via the first defecation event sensor when the wake-up sensor senses wake-up stimuli associated with defecation events of the subject. The one or more processors are further configured to associate wake-up stimuli sensed by the wake-up sensor within a second predetermined time period of the detected defecation event with defecation events of the subject.

In some embodiments, the one or more processors comprise a first processor operably coupled to the first defecation event sensor and a second processor operably coupled to the second defecation event sensor, wherein the first processor and the second processor are operably coupled to each other.

In some embodiments, the one or more processors consists of a single processor operably coupled to the first defecation event sensor and the second defecation event sensor.

In some embodiments, the first wearable device includes a smartwatch.

In some embodiments, the second wearable device includes a patch configured to be carried on the torso of the subject.

In some embodiments, the first wearable device includes at least one of the one or more processors.

In some embodiments, at least one of the one or more processors is in wireless communication with the first wearable device.

In some embodiments, the first stimuli and the second stimuli are different types of stimuli.

In some embodiments, the first stimuli and the second stimuli are the same type of stimuli.

According to another embodiment of the present disclosure, a method for training one or more processors operably coupled to a first wearable device to detect defecation events of a subject includes: sensing, by a first defecation event sensor carried by the first wearable device, one or more first stimuli; sensing, by a second defecation event sensor carried by a second wearable device, one or more second stimuli; determining, by the one or more processors, occurrence of a detected defecation event of the subject based on the second stimuli; and associating, by the one or more processors, first stimuli sensed by the first defecation event sensor within a predetermined time period of the detected defecation event with defecation events of the subject.

In some embodiments, the method further comprises training, by the one or more processors, a machine-learning algorithm for detecting defecation events of the subject with data indicative of first stimuli sensed by the first defecation event sensor that has been associated with defecation events of the subject.

In some embodiments, the first wearable device further includes a wake-up sensor configured to sense one or more wake-up stimuli, the first wearable device being configured to transition from a sleep mode to an active mode in which the first wearable device is configured to sense defecation events of the subject via the first defecation event sensor when the wake-up sensor senses wake-up stimuli associated with defecation events of the subject. The method further includes associating, by the one or more processors, wake-up stimuli sensed by the wake-up sensor within a second predetermined time period of the detected defecation event with defecation events of the subject. The second predetermined time period may be the same as or different from the predetermined time period.

According to another embodiment of the present disclosure, a system for training one or more processors to detect defecation events of a subject includes a wearable device configured to be carried on the body of the subject. The wearable device includes a defecation event sensor configured to sense one or more stimuli. The system further includes a mobile device configured to receive user input from the subject indicating a defecation time point at which a defecation event occurred. The system further includes one or more processors operably coupled with the defecation event sensor and the mobile device configured to associate stimuli sensed by the defecation event sensor within a predetermined time period of the defecation time point with defecation events of the subject.

In some embodiments, the one or more processors are further configured to train a machine-learning algorithm for detecting defecation events of the subject with data indicative of first stimuli sensed by the first defecation event sensor that has been associated with defecation events of the subject.

In some embodiments, the wearable device further comprises a wake-up sensor configured to sense one or more wake-up stimuli, wherein the wearable device is configured to transition from a sleep mode to an active mode in which the wearable device is configured to sense defecation events of the subject via the defecation event sensor when the wake-up sensor senses wake-up stimuli associated with defecation events of the subject. The one or more processors are further configured to associate wake-up stimuli sensed by the wake-up sensor within a second predetermined time period of the detected defecation event with defecation events of the subject. The second predetermined time period may be the same as or different from the predetermined time period.

According to yet another embodiment of the present disclosure, a method for training one or more processors operably coupled to a wearable device to detect defecation events of a subject includes sensing, by a defecation event sensor carried by the wearable device, one or more stimuli. The method further includes receiving user input via a mobile device of the subject indicating a defecation time point at which a defecation event occurred. The method further includes associating, by the one or more processors, stimuli sensed by the defecation event sensor within a predetermined time period of the defecation time point with defecation events of the subject.

In some embodiments, the method further comprises training, by the one or more processors, a machine-learning algorithm for detecting defecation events of the subject with data indicative of first stimuli sensed by the first defecation event sensor that has been associated with defecation events of the subject.

In some embodiments, the wearable device further comprises a wake-up sensor configured to sense one or more wake-up stimuli, the wearable device being configured to transition from a sleep mode to an active mode when the wake-up sensor senses wake-up stimuli associated with defecation events of the subject, the method further comprising associating, by the one or more processors, wake-up stimuli sensed by the wake-up sensor within a second predetermined time period of the detected defecation event with defecation events of the subject.

### BRIEF DESCRIPTION OF THE DRAWINGS

The above-mentioned and other advantages and objects of this invention, and the manner of attaining them, will become more apparent, and the invention itself will be better understood, by reference to the following description of embodiments of the invention taken in conjunction with the accompanying drawings, wherein:
FIG. 1 is a schematic representation of a system for sensing defecation events of a subject according to an embodiment of the present disclosure.
FIG. 2 is a perspective view of wearable device for sensing defecation events of a subject according to another embodiment of the present disclosure.
FIG. 3 is a bottom view of the wearable device of FIG. 2.
FIGS. 4A and 4B are front and rear perspective views, respectively, of a wearable device for sensing defecation events of a subject according to another embodiment of the present disclosure.
FIG. 5 is a perspective view of a wearable device for sensing defecation events of a subject according to yet another embodiment of the present disclosure.
FIG. 6 is a perspective view of a wearable device for sensing defecation events of a subject according to a further embodiment of the present disclosure.
FIG. 7 is a top view of the wearable device of FIG. 6.
FIG. 8 is a bottom view of the wearable device of FIG. 6.
FIG. 9 is a flow diagram of a method for sensing defecation events of a subject according to an embodiment of the present disclosure.
FIGS. 10-16 illustrate actions associated with another method for sensing defecation events of a subject according to an embodiment of the present disclosure.
FIG. 17 is a flow diagram of a method for training a first wearable device to sense defecation events by using a second wearable device, according to an embodiment of the present disclosure.
FIG. 18 is a flow diagram of a method for training a wearable device to sense defecation events using manual user input received via a mobile device of the subject, according to an embodiment of the present disclosure.

Corresponding reference characters indicate corresponding parts throughout the several views. Although the drawings represent embodiments of the present invention, the drawings are not necessarily to scale, and certain features may be exaggerated or omitted in some of the drawings in order to better illustrate and explain the present invention.

### DETAILED DESCRIPTION OF THE DRAWINGS

Systems and methods according to embodiments of the present disclosure facilitate sensing defecation events of subjects by sensing one or more stimuli associated with defecation events. Subjects may be participants in clinical trials for treatments for gastrointestinal diseases, such as irritable bowel syndrome (IBS), inflammatory bowel disease (IBD), and chronic constipation. Alternatively, systems and methods according to embodiments of the present disclosure may be used by individual patients for sensing and tallying defecation events, and resulting data may be reviewed by a healthcare provider when evaluating patient gastrointestinal health and/or treatments.

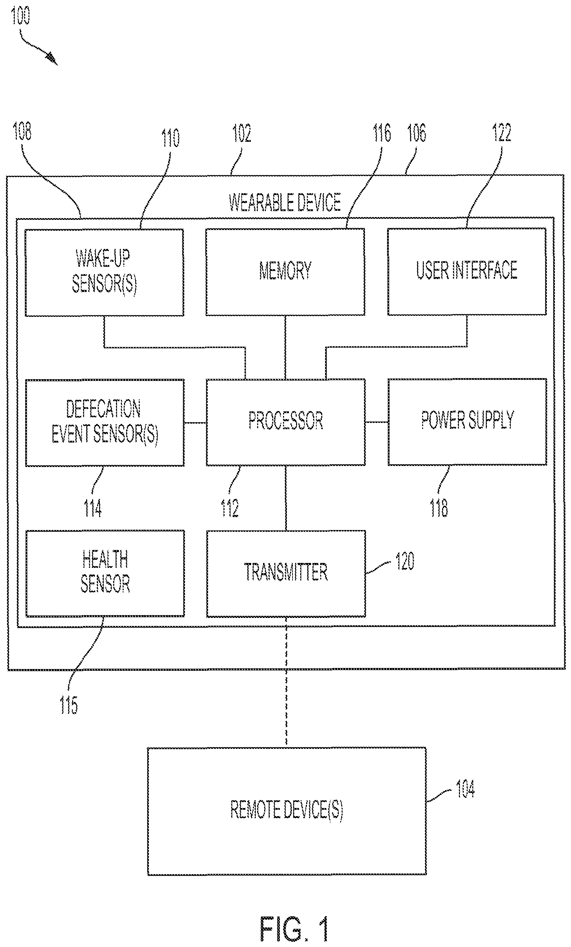

Referring now to FIG. 1, a system 100 for sensing defecation events of a subject according to an embodiment of the present disclosure is schematically illustrated. Generally, the system 100 includes a wearable device 102 that is configured to be worn by, attached to, or otherwise carried by a subject. The wearable device 102 senses one or more stimuli indicative of defecation events of the subject. The wearable device 102 is operably coupled to one or more remote devices 104 (illustratively, via wireless communication - as used in the present application, the term "operably coupled" includes wired data communication and wireless data communication, whether direct or indirect via one or more intervening devices or components, and such data communication may be continuous or intermittent), and the wearable device 102 sends data regarding defecation events to the remote device(s) 104. The remote device 104 may analyze, display, or otherwise provide the data regarding defecation events to one or more users, such as clinical trial administrators, healthcare providers, or the subject herself/himself. These aspects are described in further detail below.

With continued reference to FIG. 1, the wearable device 102 includes a base 106 that is configured to be attached to or otherwise carried by a subject. The base 106 carries an electronics assembly 108 that facilitates sensing and tallying defecation events of a subject. In the illustrated embodiment, the electronics assembly 108 includes one or more wake-up sensors 110 that sense "wake-up" stimuli preceding a defecation event. The wake-up sensor(s) 110 operably couple to a processor 112 (illustratively, via wired communication). When the wake-up sensor(s) 110 sense one or more wake-up stimuli, the processor 112 reconfigures the device 102 from a sleep mode to an active mode. In the sleep mode, one or more components of the electronics assembly 108 may operate in a low-power consumption state (e.g., operating at a lower clock rate, at a lower voltage, or both), or be inactive or "off". In some embodiments, in the sleep mode, the processor 112 may be in a state where signals output from sensors are ignored, not processed, or not stored. In the active mode, one or more components of the electronics assembly 108 may operate in a high-power consumption state (e.g., operating at a higher clock rate, at a higher voltage, or both), or be active or "on". Illustratively, such components may include one or more defecation event sensors 114 that sense stimuli during a defecation event. In some embodiments, in the active mode, the processor 112 may be put into a state where signals output from sensors are processed and/or stored. Stated differently, the wake-up sensor(s) 110 may activate the device 102 for potential defecation events, and the defecation event sensors 114 may then be used to confirm occurrence of defecation events.

The wake-up sensor(s) 110 may take various forms. For example, the wake-up sensor(s) 110 may include one or more optical sensors and/or one or more resistive force sensors that sense when the subject removes lower-body clothing. More specifically, the optical sensor(s) sense increased light when the subject removes the lower-body clothing, and the resistive force sensor(s) sense a lack of a contact force applied by the lower-body clothing. As another example, the wake-up sensor(s) 110 may include one or more audio sensors that sense one or more corresponding sounds when the subject removes the lower-body clothing. As yet another example, the wake-up sensor(s) 110 may include one or more accelerometers and/or inertial measurement units (IMUs) that detect changes in the user's posture, e.g., associated with sitting down or standing up. The wake-up stimuli sensed by the wake-up sensors and which causes the electronic assembly 108 to switch from the inactive state to the active state may comprise a single sensed stimulus, e.g., increased light, removal of contact force from resistive force sensor, and/or sounds associated with removal of clothing. Alternatively, or in addition, the wake-up stimuli may comprise a plurality of stimulus (e.g., any of the previously-mentioned stimuli) sensed in a particular sequence, or which are grouped close in time. Specific embodiments of wearable devices including such wake-up sensors are described in further detail below.

Similarly, the defecation event sensor(s) 114 may take various forms. For example, the defecation event sensor(s) 114 may include one or more electromyogram (EMG) electrodes for sensing abdominal muscle electrical signals of the subject, which may indicate contraction of those muscles during defecation events, e.g., during a Valsalva maneuver. As another example, the defecation event sensor(s) 114 may include one or more electrocardiogram (ECG) electrodes and/or one or more photoplethysmography (PPG) sensors for sensing cardiac electrical signals of the subject, which may indicate a decrease and subsequent increase in heart rate during a Valsalva maneuver. As another example, the defecation event sensor(s) 114 may include one or more mechanomyogram (MMG) sensors for sensing low frequency vibrations of the abdominal muscles of the subject, which may indicate contraction of those muscles during defecation events. As another example, the defecation event sensor(s) 114 may include one or more inertial measurement units (IMUs) for sensing changes in posture of the subject, more specifically a change to a sitting posture before a defecation event and/or a change to a standing posture after a defecation event. As another example, the defecation event sensor(s) 114 may include one or more audio sensors for sensing sounds emanating from the bowels of the subject, toilet flushing sounds, and/or sounds associated with flatus. As another example, the defecation event sensor(s) 114 may include one or more gas sensors configured to sense flatus. As another example, the defecation event sensor(s) 114 may include one or more temperature sensors for sensing bowel temperature changes that may occur before or during a defecation event. As another example, the defecation event sensor(s) 114 may include one or more optical sensors and/or one or more resistive force sensors that sense when the subject removes lower-body clothing. Specific embodiments of wearable devices including such defecation event sensors are described in further detail below.

With continued reference to FIG. 1, in the illustrated embodiment, the electronics assembly 108 further includes one or more health sensors 115 that sense stimuli associated with the health of the subject. The health sensor(s) 115 may take various forms. For example, a health sensor 115 may be a blood sensor that detects blood in stool of the subject, which may indicate one or more gastrointestinal diseases. More specifically, a blood sensor may be a solid-state vapor detection sensor that detects one or more volatile organic compounds (VOCs) that create a "metallic smell" (for example, hexanal, heptanal, octanal, nonanal, decanal, and/or 1-octen-3-one, formed by a reaction of ions in the blood and lipids in the stool). In some embodiments, the health sensor(s) 115 may normally be in a sleep mode, and the health sensor(s) 115 may be reconfigured to an active mode when one or more of the defecation event sensors 114 detects a defecation event. Alternatively, the health sensor(s) 115 may normally be in a sleep mode, and the health sensor(s) 115 may be reconfigured to an active mode when one or more of the wake-up sensors 110 sense one or more wake-up stimuli.

With further reference to FIG. 1, the processor 112 may be any device or component capable of executing stored software and/or firmware code that, when executed by the processor 112, causes the wearable device 102 to perform the functions described herein. The processor 112 may be, for example, an application-specific integrated circuit (ASICs), a field-programmable gate array (FPGA), a digital signal processor (DSP), hardwired logic, combinations thereof, or the like.

The processor 112 operably couples to a memory 116 (illustratively, via wired communication) for storing data regarding defection events and/or subject health. Such data may include, for example, defecation event time, event length, wake-up sensor(s) 110 and/or defecation event sensors 114 that sensed the event, wake-up sensor(s) 110 and/or defecation event sensors 114 that did not sense the event, data received from the health sensor(s) 115, and the like. The memory 116 may be any suitable computer readable medium that is accessible by the processor 112. The memory 116 may be a single storage device or multiple storage devices, may be located internally or externally to the processor 112, and may include both volatile and non-volatile media. The memory 116 may be, for example, a random-access memory (RAM), a read-only memory (ROM), an electrically erasable programmable ROM (EEPROM), a flash memory, a magnetic storage device, an optical disk storage, or any other suitable medium which is capable of storing data and which is accessible by the processor 112.

The processor 112 also operably couples to a power supply 118 (illustratively, via wired communication) for providing power to the various components of the electronics assembly 108, including the wake-up sensor(s) 110, the defecation event sensor(s) 114, and the health sensor(s) 115. The power supply 118 may be, for example, one or more rechargeable batteries, one or more inductive/wireless power receivers, or the like.

With continued reference to FIG. 1, the processor 112 also operably couples to a user interface 122 (illustratively, via wired communication). The user interface 122 is operative to receive one or more user inputs (for example, to manually confirm occurrence of a defecation event) and/or to display data, information, and/or prompts generated by the system 100. The user interface 122 may include at least one input device for receiving user inputs. The user interface 122 may include a graphical user interface (GUI) having a touchscreen display operative to display data and receive user inputs. Alternatively, the user interface 122 may include a non-touchscreen display, a keyboard, a keypad, a microphone, a speaker, combinations thereof, or the like.

The processor 112 further operably couples to a transmitter 120 (illustratively, via wired communication) for wirelessly transmitting information, such as defecation event information and/or subject health data stored by the memory 116, to the remote device(s) 104. The transmitter 120 may be, for example, a Bluetooth transmitter, an IEEE 802.11 transmitter, a cellular communication transmitter, a near-field communication transmitter, or the like. The transmitter 120 may be continuously coupled or intermittently coupled to the remote device(s) 104. A transceiver (not shown) may be used instead of the transmitter 120 to facilitate providing information from the remote device(s) 104 to the wearable device 102. Such information, may include, for example, software updates.

The remote device(s) 104 may be, for example, mobile devices, such as smartphones, smartwatches, or tablet devices, personal computers, remote computers or databases, or the like. In clinical trial settings, the remote device(s) 104 may be capable of analyzing defecation event data and/or subject health data received from various wearable devices 102 and evaluating efficacy of one or more treatments provided to subjects using the wearable devices 102. In other settings, the remote device(s) 104 may include or be operably coupled to one or more displays for providing defecation event data and/or subject health data to a user, such as a healthcare provider or the subject using the wearable device 102.

The system 100, more specifically the wearable device 102, may be modified in various manners. For example, the transmitter 120 may couple to the remote device(s) 104 via wired communication, or the processor 112 may operably couple to one or more of the other components of the electronics assembly 108 via wireless communication. Similarly, in some embodiments the wearable device 102 may lack a processor 112, and the sensor(s) 110, 114, 115 may instead be operably coupled to a processor of a remote device 104, such as a processor of a smartphone. In some embodiments, wearable device 102 may not include a user interface 122, and rely instead on wireless communication with remote device(s) 104 to convey information to and/or receive instructions from a user. As a further example, in some embodiments there may be two or more wearable devices 102 that are each operably coupled to remote device 104. For instance, there may be a first wearable device 102 that takes the form of a smartwatch and a second wearable device 102 that takes the form of a patch configured to be carried on the torso of a subject, wherein both wearable devices are operably coupled to a remote device 104 (e.g., a smartphone). In such embodiments, all wearable devices may comprise similar sensors 110, 114, and/or 115, or the wearable devices may comprise different sensors 110, 114, and/or 115. As a specific example, a first wearable device 102 taking the form of a smartwatch may include audio sensors and accelerometers / motion sensors that serve as defecation event sensors, while a second wearable device 102 taking the form of a torso patch may comprise other types of defecation event sensors (e.g., gas sensors, mechanomyogram sensors, and/or electromyogram sensors). Since all wearable devices are operably coupled to remote device(s) 104, remote device(s) 104 may use inputs from all operably coupled wearable devices to detect defecation events.

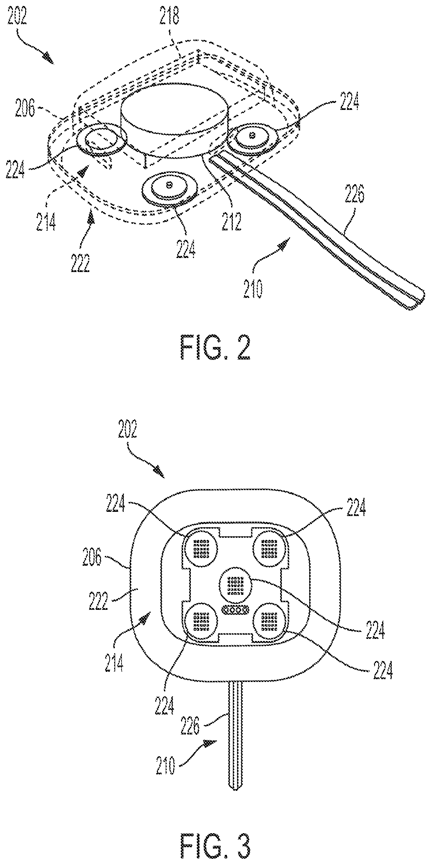

Referring now to FIGS. 2 and 3, a wearable device 202 according to an embodiment of the present disclosure is illustrated. The wearable device 202 is a more specific embodiment of the wearable device 102 described above. Accordingly, the wearable device 202 includes some of the same components and operates in a similar manner to the wearable device 102 described above. The wearable device 202 also operatively couples to one or more remote devices 104 as described above. The wearable device 202 includes a base or housing 206 that is configured to be detachably worn by a subject. More specifically, the base 206 is a patch that can be adhesively secured to the torso of a subject. A patient-facing side 222 of the base 206 can be adhesively secured to the subject. The patient-facing side 222 of the base 206 also includes one or more defecation event sensors 214, more specifically a plurality of electromyogram electrodes 224 for sensing abdominal muscle electrical signals of the subject. The base 206 also carries one or more wake-up sensors 210, more specifically an elongated optical sensor 226 that is configured to be disposed under lower-body clothing and sense when the subject removes the lower-body clothing. The base 206 internally carries a processor 212 and a power supply 218. In some embodiments, the base 206 may carry additional sensors, such as an inertial measurement unit, a gas sensor, a mechanomyogram sensor, or any of the other sensors contemplated herein.

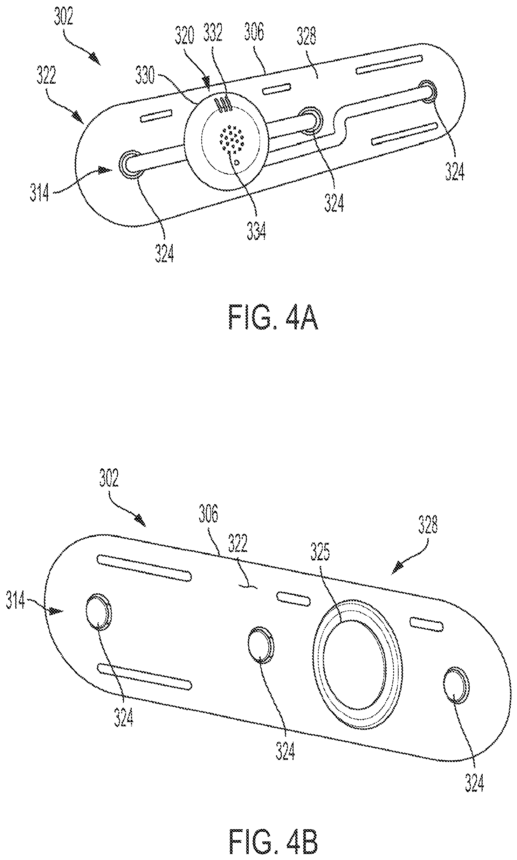

Referring to FIGS. 4A and 4B, a wearable device 302 according to another embodiment of the present disclosure is illustrated. The wearable device 302 is another more specific embodiment of the wearable device 102 described above. Accordingly, the wearable device 302 includes some of the same components and operates in a similar manner to the wearable device 102 described above. The wearable device 302 also operatively couples to one or more remote devices 104 as described above. The wearable device 302 includes a base 306 that is configured to be detachably worn by a subject. More specifically, the base 306 is a patch that can be adhesively secured to the torso of a subject. A patient-facing side 322 of the base 306 can be adhesively secured to the subject. The patient-facing side 322 of the base 306 also includes one or more defecation event sensors 314, more specifically a plurality of electromyogram electrodes 324 for sensing abdominal muscle electrical signals of the subject and a mechanomyogram sensor 325 (FIG. 4B) for sensing abdominal muscle movement signals of the subject. An opposite side 328 of the base 306 detachably carries a hub 330 (FIG. 4A) that operatively couples to the defecation event sensor(s) 314. The hub 330 carries a processor (not shown), a memory (not shown), and power supply (not shown). The hub 330 may be detached from the base 306 to facilitate recharging the power supply. The hub 330 also carries a user interface 320 (FIG. 4A), more specifically one or more lights 332 and a speaker 334 for providing information to the subject. In some embodiments, the base 306 may carry additional sensors, such as an optical sensor, an audio sensor, an inertial measurement unit, a gas sensor, or any of the other sensors contemplated herein.

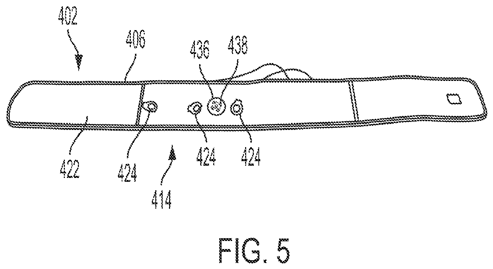

Referring to FIG. 5, a wearable device 402 according to yet another embodiment of the present disclosure is illustrated. The wearable device 402 is yet another more specific embodiment of the wearable device 102 described above. Accordingly, the wearable device 402 includes some of the same components and operates in a similar manner to the wearable device 102 described above. The wearable device 402 also operatively couples to one or more remote devices 104 as described above. The wearable device 402 includes a base 406 that is configured to be detachably worn by a subject. More specifically, the base 406 is a belt that is extendable around and securable to the torso of the subject. The patient-facing side 422 of the base 406 also includes one or more defecation event sensors 414, more specifically a plurality of electromyogram electrodes 424, an inertial measurement unit 436, and an mechanomyogram sensor 438. In some embodiments, the base 406 may carry additional sensors, such as an optical sensor, an audio sensor, or any of the other sensors contemplated herein.

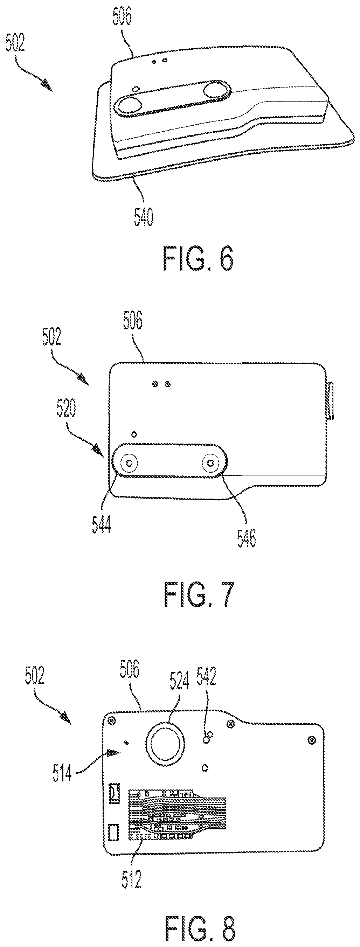

Referring now to FIGS. 6-8, a wearable device 502 according to yet another embodiment of the present disclosure is illustrated. The wearable device 502 is yet another more specific embodiment of the wearable device 102 described above. Accordingly, the wearable device 502 includes some of the same components and operates in a similar manner to the wearable device 102 described above. The wearable device 502 also operatively couples to one or more remote devices 104 as described above. The wearable device 502 includes a base or housing 506 that is configured to be detachably worn by a subject. More specifically, the base 506 carries a patch 540 (FIG. 6) that can be adhesively secured to the torso of a subject. The base 506 also carries one or more defecation event sensors 514 (FIG. 8). More specifically, the base 506 carries a mechanomyogram sensor 524 for sensing abdominal muscle movement signals of the subject and a temperature sensor 542 for sensing bowel temperature changes. The base 506 internally carries a processor 512 (FIG. 8) and a battery (not shown). The base 506 further carries a user interface 520 (FIG. 7). The user interface 520 includes a first input 544 that is actuatable to reconfigure the device 502 from a sleep mode to an active mode. The user interface 520 further includes a second input 546 that is actuatable to reconfigure the device 502 from the active mode to the sleep mode. In some embodiments, the base 506 may carry additional sensors, such as an optical sensor, an inertial measurement unit, an audio sensor, a gas sensor, a electromyogram sensor, or any of the other sensors contemplated herein.

Systems according embodiments of the present disclosure may determine occurrence of defecation events in various manners. For example, in some embodiments systems may determine occurrence of defecation events if at least a certain number of the defecation event sensors 114 sense corresponding stimuli (as a more specific example, if the majority of the defecation event sensors 114 sense corresponding stimuli). In other embodiments, systems may determine occurrence of defecation events if all of the defecation event sensors 114 sense corresponding stimuli. In some embodiments, systems may use machine learning to increase accuracy for determining occurrence of defecation events for specific subjects. More specifically, some systems may recognize that one or more specific stimuli are consistently sensed before or during defecation events for specific subjects, and the system may more heavily weight information received from corresponding sensors when determining occurrence of defecation events. In some embodiments, systems may determine occurrence of defecation events if the defecation event sensors 114 sense corresponding stimuli according to a certain sequence or algorithm. Similarly, in some embodiments, systems may reconfigure devices from sleep modes to active modes if the wake-up sensors 110 sense corresponding stimuli according to a certain sequence or algorithm.

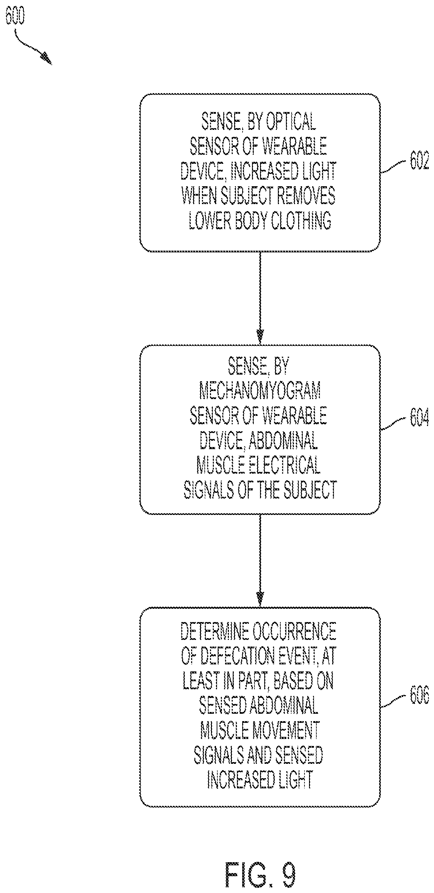

Referring to FIG. 9, a method 600 for sensing defecation events of a subject according to an embodiment of the present disclosure is illustrated. Before beginning the method 600, a wearable device, such as any of the wearable devices contemplated herein, is attached to the torso of a subject. The method 600 begins at block 602 by sensing, by an optical sensor of the wearable device, increased light when the subject removes lower-body clothing. The method 600 continues at block 604 by sensing, by a mechanomyogram sensor of the wearable device, abdominal muscle movement signals of the subject. The method 600 concludes at block 606 by determining occurrence of the defecation event, at least in part, based on the sensed abdominal muscle movement signals of the subject and the sensed increased light when the subject removes the lower-body clothing.

The method 600 may be modified in various manners and/or include various additional actions. For example, the method 600 may further include reconfiguring the wearable device from a sleep mode, in which the mechanomyogram sensor is inactive, to an active mode, in which the mechanomyogram sensor is configured to sense the abdominal muscle movement signals of the subject, based on the sensed increased light when the subject removes the lower-body clothing. As another example, the method 600 may further include sensing, by a gas sensor of the wearable device, flatus, and the determination that the defecation event has occurred may be based at least in part on the sensed flatus. As yet another example, the method 600 may further include sensing, by an inertial measurement unit of the wearable device, a standing motion by the subject after sensing the flatus, and the determination that the defecation event has occurred may be based at least in part on the sensed standing motion. As yet another example, the method 600 may further include sensing, by an inertial measurement unit of the wearable device, a sitting motion by the subject before sensing the abdominal muscle movement signals of the subject, and the determination that the defecation event has occurred may be based at least in part on the sensed sitting motion. As another example, the method 600 may be repeated to determine a plurality of defecation events over a certain time period, such as multiple days, weeks, months, or years. As an alternative example, the sensing of abdominal muscle movement signals in block 604 may precede the sensing of increased light in block 602.

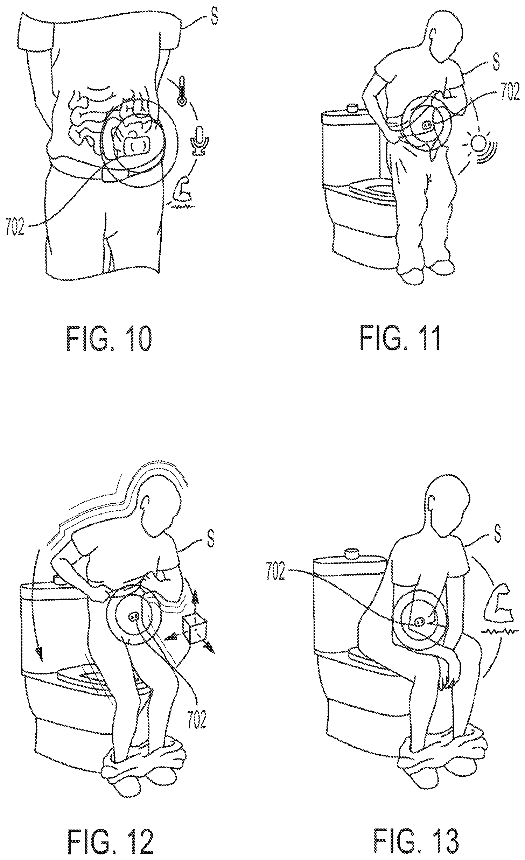

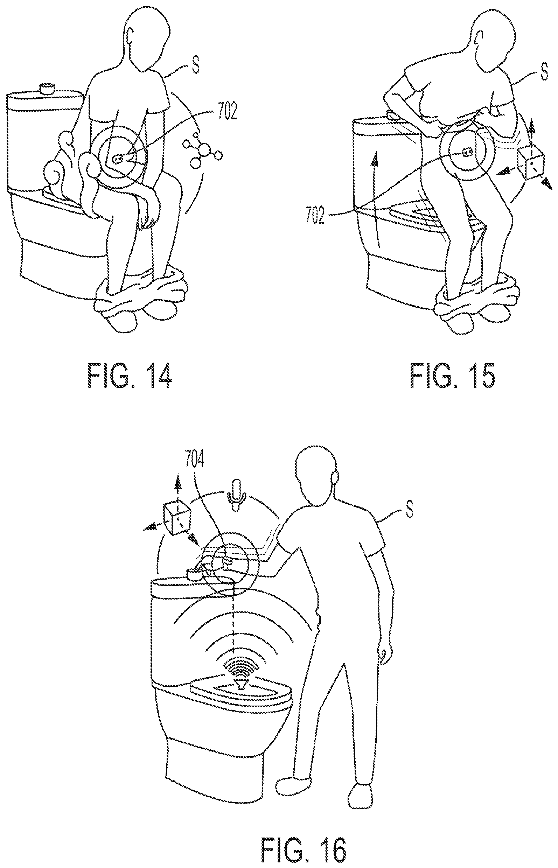

FIGS. 10-16 illustrate actions associated with another method for sensing defecation events of a subject according to an embodiment of the present disclosure. Before beginning the method, a wearable device 702, such as any of the wearable devices contemplated herein, is attached to the torso of a subject S. As shown in FIG. 10, the method begins by (A) sensing, by a mechanomyogram sensor (not shown) of the wearable device 702, abdominal muscle movement signals of the subject S; (B) sensing, by a temperature sensor (not shown) of the wearable device 702, bowel temperature changes of the subject; and/or (C) sensing, by an audio sensor (not shown) of the wearable device 702, sounds emanating from the bowels of the subject. As shown in FIG. 11, the method continues by sensing, by an optical sensor (not shown) of the wearable device 702, increased light when the subject S removes lower-body clothing. As shown in FIG. 12, the method next includes sensing, by an inertial measurement unit (not shown) of the wearable device 702, a sitting motion of the subject S. As shown in FIG. 13, the method continues by sensing, by the mechanomyogram sensor (not shown) of the wearable device 702, abdominal muscle movement signals of the subject S. As shown in FIG. 14, the method next includes sensing, by a gas sensor (not shown) of the wearable device 702, flatus. As shown in FIG. 15, the method continues by sensing, by the inertial measurement unit (not shown) of the wearable device 702, a standing motion of the subject S. As shown in FIG. 16, the method next includes (A) sensing, by an inertial measurement unit (not shown) of a remote device, illustratively a smart watch 704 worn by the subject S, a hand motion for flushing the toilet; and/or (B) sensing, by an audio sensor (not shown) of the remote device, toilet flushing sounds. The method concludes by determining occurrence of the defecation event, at least in part, based on one or more of the previously sensed stimuli. The method for sensing defecation events presented in FIGS. 10-16 may be modified by omitting one or more of the previously-presented steps, adding additional steps between some of the previously-presented steps, and/or by rearranging the order of the presented steps. For example, in some embodiments, the method may not include sensing bowel temperature changes and/or sounds emanating from the bowels of the subject (as shown in FIG. 10), but instead may begin with sensing of increased light when the subject S removes lower-body clothing (as shown in FIG. 11). In some embodiments, the method may omit the sensing of flatus (as shown in FIG. 14). In yet other embodiments, the method may omit the sensing of changes in posture by the inertial measurement unit, as shown in FIGS. 13 and 15. Any sequence of all or any subset of the presented steps may be used to determine the occurrence of the defecation event.

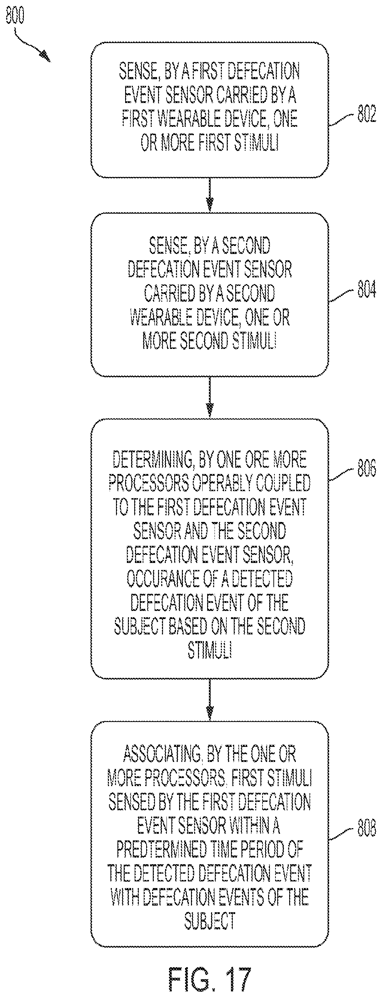

The sensitivity and specificity of the systems and methods described herein for detecting defecation events of subjects may be improved using machine-learning techniques. Referring to FIG. 17, a method 800 for training one or more processors to detect defecation events according to an embodiment of the present disclosure is illustrated. Before beginning the method 800, a first wearable device, such as a smartwatch or any of the wearable devices contemplated herein, and a second wearable device, such as any of the wearable devices contemplated herein, may be provided to a subject for attachment to the body of the subject. Both wearable devices may take the form of wearable device 102 described herein (see, e.g., FIG. 1). The first wearable device may comprise a first defecation event sensor 114 configured to sense one or more first stimuli, while the second wearable device may comprise a second defecation event sensor 114 configured to sense one or more second stimuli. The first wearable device and the second wearable device are operably coupled to one or more processors, either wirelessly or through a wired connection in any of the manners contemplated herein. In some embodiments, the one or more processors may comprise a first processor operably coupled to the first wearable device (e.g., embedded within or in wireless communication with the first wearable device) and a second processor operably coupled to the second wearable device (e.g., embedded within or in wireless communication with the second wearable device). In such embodiments, the first processor may be operably coupled to the second processor such that the two processors may work together to implement method 800. In some embodiments, the one or more processors may consist of a single processor operably coupled to both the first wearable device and the second wearable device. This single processor may reside on the first wearable device, on the second wearable device, or on a remote device 104 (e.g., a smartphone) operably coupled with both wearable devices.

The method 800 begins at block 802 by sensing, by one or more first defecation event sensors carried by the first wearable device, one or more first defecation stimuli. The method 800 continues at block 804 by sensing, by one or more second defecation events sensors carried by the second wearable device, one or more second stimuli associated with one or more defection events of the subject. The sensed stimuli (by both the first defecation event sensors and the second defecation event sensors) may be any of the stimuli contemplated herein. In some embodiments, the first defecation stimuli and the second defecation stimuli may be different types of stimuli. For example, the second defecation event sensor may be an EMG electrode and/or MMG sensor and the second stimuli may be abdominal muscle electrical signals and/or abdominal muscle movement signals of the subject, and the first defecation event sensor may be an inertial measurement unit and the first stimuli may be motion of the subject. In other embodiments, the first defecation stimuli and the second defecation stimuli may be the same type of stimuli. The method 800 continues at block 806 by determining, by the one or more processors, occurrence of a detected defecation event of the subject based on the second stimuli. This determination may be accomplished through any of the methods discussed herein. The method 800 continues at block 808 by associating, by the one or more processors, first stimuli sensed by the first defecation event sensor within a predetermined time period of the detected defecation event (e.g., within 60, 120, 180, and/or 240 seconds before and/or after the detected defecation event) with defecation events of the subject. The one or more processors may then train a machine learning algorithm for detecting defecation events of the subject with data indicative of first stimuli sensed by the first defecation event sensor that has been associated with defecation events of the subject.

Method 800 may be useful for training a machine learning algorithm implemented on the one or more processors to use the first stimuli sensed by the first defecation event sensors to detect defecation events of the subject. In particular, method 800 may be useful in situations where the second defecation event sensors are initially capable of detecting defecation events with greater sensitivity and/or specificity than the first defecation event sensors, but it is desirable to eventually detect defecation events using only or primarily the first defecation event sensors.

As an illustrative and non-limiting example, the second wearable device may be patch configured to be secured to the torso of a subject, as described herein. This patch may comprise MMG sensors, EMG sensors, ECG sensors, optical sensors, gas sensors, and/or other sensors as described herein. The first wearable device may be a smartwatch configured to be worn on the wrist of a subject. The smartwatch may comprise additional or different sensors, such as an accelerometer / inertial measurement unit (IMU) and/or an audio sensor. The second wearable device may be initially capable of detecting defecation events with greater specificity and sensitivity than the first wearable device but may be more intrusive and/or inconvenient for the subject to wear for an extended period of time. Thus, it may be desirable to eventually train the one or more processors to use only the first wearable device (i.e., the smartwatch) to detect defecation events without the aid of the second wearable device. To accomplish this training, when the second wearable device detects a defecation event using the techniques described herein, it may instruct the one or more processors to associate the movements and/or audio signals recorded by the smartwatch in the recent past (e.g., within 60 or 120 seconds before or after the detected defecation event) with defecation. In this way, the second wearable device provides a ground truth label that allows the smartwatch to discern movements and/or audio signals associated with defecation with movements and/or audio signals that are not associated with defecation. Over time, as the wearable patch trains the one or more processors, the processor(s) can use stimuli sensed by the smartwatch to predict and/or detect defecation with greater accuracy. Eventually, when the smartwatch (or mobile device) is fully trained, the subject may stop wearing the wearable patch and rely solely on the smartwatch to detect defecation events.

The training of the machine-learning algorithm at the one or more processors may be accomplished using any known machine learning technique. For instance, the one or more processors may employ a neural network having a plurality of layers of nodes to predict or detect defection events based on stimuli sensed by the first defecation event sensor. The sensitivity and specificity of such a neural network may be improved by adjusting the weights associated with such layers of nodes using ground truth data that provides examples of first stimuli associated with defecation events, and examples of first stimuli that are not associated with defecation events. Such ground truth data may be provided by the second defecation event sensors on the second wearable device, as described herein. The weights within the neural network may be adjusted using an iterative training procedure that compares a predicted output based on certain first stimuli with a ground truth label provided by the second wearable device that indicates whether such first stimuli is or is not associated with a defecation event. If the neural network's prediction does not match the ground truth label, the weights may be adjusted according to a loss function to improve the match between the network's prediction and the ground truth label. In this way, by providing ground truth labels that indicate whether certain first stimuli is or is not associated with a defecation event, the weights of the neural network may be adjusted to improve the network's detection of defecation events based on first stimuli only.

The method 800 may be modified in various manners and/or include various additional actions. For example, the one or more processors may be trained to determine occurrence of defecation events if one or more defecation event sensors of the first wearable device sense corresponding stimuli according to a certain sequence or algorithm. As another example, the first wearable device may further comprise a wake-up sensor configured to sense one or more wake-up stimuli. The first wearable device may be further configured to transition from a sleep mode to an active mode in which the first wearable device is configured to sense defecation events of the subject via the first defecation event sensor when the wake-up sensor senses wake-up stimuli associated with defecation events of the subject. Method 800 may be modified by having the one or more processors associate wake-up stimuli sensed by the wake-up sensor within a second predetermined time period of the detected defecation event (e.g., within 60, 120, 180, and/or 240 seconds before and/or after the detected defecation event) with defecation events. The second predetermined time period may be the same as the predetermined time period, or it may be different from the predetermined time period. In this way, the one or more processors may be trained to not only detect defecation events with greater sensitivity and specificity, but to also wake up from a sleep mode to an active mode to detect such defecation events with greater accuracy. This may be helpful in conserving power used by the one or more processors and/or the first wearable device.

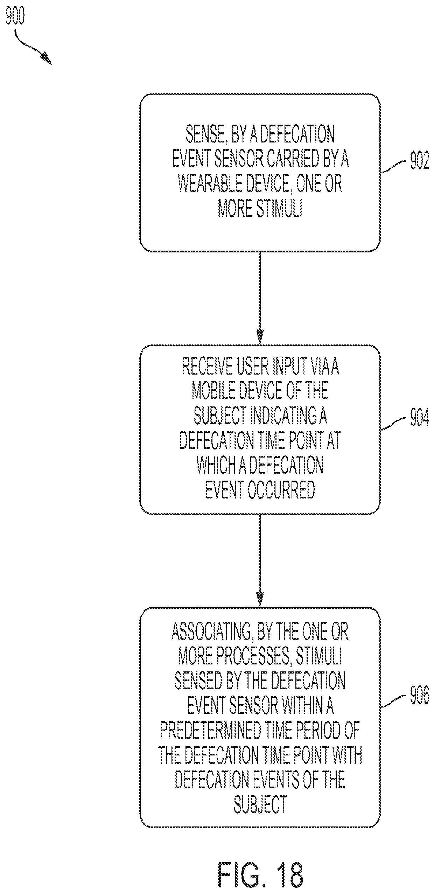

Referring to FIG. 18, another method 900 for training one or more processors to detect defecation events according to an embodiment of the present disclosure is illustrated. Before beginning method 900, a wearable device, such as any of the wearable devices contemplated herein, may be provided to a subject for attachment to the body of the subject. The wearable device may take the form of wearable device 102 described herein (see, e.g., FIG. 1), and may comprise a defecation event sensor 114 configured to sense one or more stimuli. The wearable device, and the defecation event sensors provided therein, may be operably coupled to one or more processors. The one or more processors may be provided on the wearable device or may be in wireless communication with the wearable device. For instance, the one or more processors may reside on a remote device 104 (e.g., a smartphone).

The method 900 begins at block 902 by sensing, by the defecation event sensor of the wearable device, one or more stimuli. The method 900 continues at block 904 by receiving user input, via a mobile device of the subject (e.g., remote device 104) indicating a defecation time point at which a defecation event occurred. The user input may comprise manual input (e.g., actuation of a physical button and/or virtual button on a touch screen, a voice input) from the subject that a defecation event occurred at the time the user provided the input. Alternatively, or in addition, the user input may comprise manual input from the subject indicating that a defecation event occurred at a specific time point in the past. Alternatively, or in addition, the user input may comprise manual input from the subject indicating that a defecation event is about to occur (e.g., the subject is about to have a bowel movement).

The method 900 continues at block 906 by associating, by the one or more processors, stimuli sensed by the defecation event sensor within a predetermined time period of the defecation time point received from the subject (e.g., within 60, 120, 180, and/or 240 seconds before and/or after the defecation time point) with defecation events. The one or more processors may then train a machine learning algorithm for detecting defecation events of the subject with data indicative of first stimuli sensed by the first defecation event sensor that has been associated with defecation events of the subject. In this way, a machine learning algorithm implemented by the one or more processors for detecting defecation events based on stimuli sensed by the defecation event sensor may be trained using ground truth data manually provided by the subject. Such training may be implemented using any of the techniques described herein.

While this invention has been shown and described as having preferred designs, the present invention may be modified within the spirit and scope of this disclosure. This application is therefore intended to cover any variations, uses or adaptations of the invention using its general principles. Further, this application is intended to cover such departures from the present disclosure as come within known or customary practice in the art to which this invention pertains.

The claims of the parent application are reproduced immediately below as clauses. These clauses define preferred embodiments. The applicant reserves the right to pursue protection for subject-matter contained in the parent application as filed, either in the present divisional application or in a further application divided from the present divisional application.
1. A system for sensing defecation events of a subject, the system comprising:
   a wearable device configured to be carried on a torso of the subject, the wearable device being operable in a sleep mode and an active mode, the wearable device comprising:
      a wake-up sensor configured to sense a first stimulus, and
      a mechanomyogram sensor configured to sense abdominal muscle movement signals of the subject; and
   a processor operably coupled to the wake-up sensor and the mechanomyogram sensor, the processor configured to switch the wearable device from the sleep mode to the active mode based on the first stimulus sensed by the wake-up sensor, and in the active mode the wearable device is configured to communicate with the processor to determine occurrence of defecation events of the subject based on abdominal muscle movement signals sensed by the mechanomyogram sensor.
2. The system of clause 1, wherein the abdominal muscle movement signals of the subject is a second stimulus, the system further comprising a third sensor operably coupled to the processor and configured to sense a third stimulus, and in the active mode the processor is configured to determine occurrence of defecation events of the subject based on abdominal muscle movement signals sensed by the mechanomyogram sensor and the third stimulus sensed by the third sensor.
3. The system of clause 2, wherein the third sensor comprises a gas sensor disposed in the wearable device and configured to sense flatus.
4. The system of clause 2, wherein the third sensor is an audio sensor configured to sense toilet flushing sounds.
5. The system of clause 2, wherein the third sensor is an electromyogram electrode configured to sense muscle electrical signals of the subject.
6. The system of clause 2, wherein the third sensor is an inertial measurement unit configured to sense a change in posture of the subject.
7. The system of any of clauses 1-6, wherein the wearable device further comprises a patch configured to be carried on the torso of the subject, the patch carrying the wake-up sensor and the mechanomyogram sensor.
8. The system of clause 7, wherein the patch further carries the processor.
9. The system of any of clauses 1-6, wherein the wearable device further comprises a belt configured to extend around the torso of the subject, the belt carrying the wake-up sensor and the mechanomyogram sensor.
10. The system of clause 9, wherein the belt further carries the processor.
11. The system of any of clauses 1-10, wherein the wake-up sensor comprises one of an optical sensor and a resistive force sensor configured to sense when the subject removes lower-body clothing.
12. The system of any of clauses 1-11, wherein the wearable device further comprises a health sensor configured to sense a health stimulus associated with health of the subject.
13. The system of clause 12, wherein the health sensor comprises a blood sensor configured to sense blood in stool of the subject.
14. The system of clause 13, wherein the blood sensor comprises a solid-state vapor detection sensor configured to sense one or more volatile organic compounds.
15. A system for sensing defecation events of a subject, the system comprising:
   a wearable device configured to be carried on a torso of the subject, the wearable device comprising:
      a mechanomyogram sensor configured to sense abdominal muscle movement signals of the subject;
      a gas sensor configured to sense flatus; and
   a processor operably coupled to the mechanomyogram sensor and the gas sensor, the processor configured to determine occurrence of defecation events of the subject based on abdominal muscle movement signals sensed by the mechanomyogram sensor and flatus sensed by the gas sensor.
16. The system of clause 15, wherein the processor is configured to determine occurrence of defecation events of the subject based on a sequence of events comprising one of the abdominal muscle movement signals sensed by the mechanomyogram sensor and the flatus sensed by the gas sensor preceding the other of the abdominal muscle movement signals sensed by the mechanomyogram sensor and the flatus sensed by the gas sensor.
17. The system of any of clauses 15-16, wherein the wearable device further comprises a base carrying the mechanomyogram sensor, the gas sensor, and the processor.
18. A system for sensing defecation events of a subject, the system comprising:
   a wearable device configured to be carried on a torso of the subject and under lower-body clothing worn by the subject, the wearable device comprising
      an optical sensor configured to sense increased light when the subject removes the lower-body clothing; and
   a processor operably coupled to the optical sensor, the processor configured to determine occurrence of defecation events based at least in part on the increased light sensed by the optical sensor.
19. The system of clause 18, wherein the wearable device is operable in an active mode and a sleep mode, and the processor is configured to switch the wearable device from the sleep mode to the active mode upon determining removal of the lower-body clothing by the subject in response to the increased light sensed by the optical sensor.
20. The system of clause 19, wherein the optical sensor is a first sensor configured to sense light as a first stimulus, wherein the wearable device further comprises a second sensor configured to sense a second stimulus when the wearable device is in the active mode, the second stimulus being different than the first stimulus, and wherein the processor is operably coupled to the second sensor, the processor being configured to determine occurrence of defecation events of the subject based on signals received from the second sensor.
21. The system of clause 19, wherein the optical sensor is a first sensor configured to sense light as a first stimulus, wherein the wearable device further comprises a second sensor configured to sense a second stimulus when the wearable device is in the active mode, the second stimulus being different than the first stimulus, and wherein the processor is operably coupled to the second sensor, the processor being configured to determine occurrence of defecation events of the subject based on signals received from the first sensor and the second sensor.
22. The system of any of clauses 20-21, wherein the second sensor is a mechanomyogram sensor.
23. The system of any of clauses 18-22, wherein the wearable device further comprises a patch configured to be carried on the torso of the subject, the patch carrying the optical sensor and the processor.
24. The system of any of clauses 18-22, wherein the wearable device further comprises a belt configured to extend around the torso of the subject, the belt carrying the optical sensor and the processor.
25. A method for sensing defecation events of a subject, the method comprising:
   sensing, by an optical sensor of a wearable device carried on a torso of the subject, increased light when the subject removes lower-body clothing;
   sensing, by a mechanomyogram sensor of the wearable device, abdominal muscle movement signals of the subject; and
   determining occurrence of the defecation event based, at least in part, on the sensed increased light when the subject removes the lower-body clothing and the sensed abdominal muscle movement signals of the subject.
26. The method of clause 25, further comprising sensing, by a gas sensor of the wearable device, flatus, and wherein the determination that the defecation event has occurred is based at least in part on the sensed flatus.
27. The method of any of clauses 25-26, further comprising sensing, by an inertial measurement unit of the wearable device, a sitting motion by the subject before sensing the abdominal muscle movement signals of the subject, and wherein the determination that the defecation event has occurred is based at least in part on the sensed sitting motion.
28. The method of any of clauses 25-27, further comprising sensing, by an inertial measurement unit of the wearable device, a standing motion by the subject after sensing the flatus, and wherein the determination that the defecation event has occurred is based at least in part on the sensed standing motion.
29. The method of any of clauses 25-28, further comprising sensing a plurality of defecation events of the subject over a time period.
30. The method of clause 29, wherein sensing each of the plurality of defecation events of the subject comprises:
   sensing, by the mechanomyogram sensor of the wearable device, abdominal muscle movement signals of the subject;
   sensing, by the optical sensor, increased light when the subject removes lower-body clothing; and
   determining occurrence of each of the plurality of defecation events based, at least in part, on the sensed abdominal muscle movement signals of the subject and the sensed increased light when the subject removes the lower-body clothing.
31. The method of any of clauses 25-30, wherein sensing, by the optical sensor, the increased light when the subject removes the lower-body clothing precedes sensing, by the mechanomyogram sensor, the abdominal muscle movement signals of the subject.
32. The method of any of clauses 25-30, wherein sensing, by the mechanomyogram sensor, the abdominal muscle movement signals of the subject precedes sensing, by the optical sensor, the increased light when the subject removes the lower-body clothing.
33. The method of any of clauses 25-30, further comprising reconfiguring the wearable device from a sleep mode to an active mode based on the sensed increased light when the subject removes the lower-body clothing, in the sleep mode the mechanomyogram sensor being inactive, and in the active mode the mechanomyogram sensor being configured to sense the abdominal muscle movement signals of the subject.
34. A system for training one or more processors to detect defecation events of a subject, the system comprising:
   a first wearable device, wherein the first wearable device is configured to be carried on a body of the subject, the first wearable device comprising a first defecation event sensor configured to sense one or more first stimuli;
   a second wearable device configured to be carried on the body of the subject, the second wearable device comprising a second defecation event sensor configured to sense one or more second stimuli; and
   one or more processors operably coupled to the first defecation event sensor and the second defecation event sensor and configured to determine occurrence of a detected defecation event of the subject based on the one or more second stimuli, and to associate first stimuli sensed by the first defecation event sensor within a predetermined time period of the detected defecation event with defecation events of the subject.
35. The system of clause 34, wherein the one or more processors are further configured to train a machine-learning algorithm for detecting defecation events of the subject with data indicative of first stimuli sensed by the first defecation event sensor that has been associated with defecation events of the subject.
36. The system of any of clauses 34-35, wherein:
   the first wearable device further comprises a wake-up sensor configured to sense one or more wake-up stimuli,
   the first wearable device is configured to transition from a sleep mode to an active mode in which the first wearable device is configured to sense defecation events of the subject via the first defecation event sensor when the wake-up sensor senses wake-up stimuli associated with defecation events of the subject; and
   the one or more processors are further configured to associate wake-up stimuli sensed by the wake-up sensor within a second predetermined time period of the detected defecation event with defecation events of the subject.
37. The system of any of clauses 34-36, wherein the one or more processors comprise a first processor operably coupled to the first defecation event sensor and a second processor operably coupled to the second defecation event sensor, wherein the first processor and the second processor are operably coupled to each other.
38. The system of any of clauses 34-36, wherein the one or more processors consists of a single processor operably coupled to the first defecation event sensor and the second defecation event sensor.
39. The system of any of clauses 34 to 38, wherein the first wearable device comprises a smartwatch.
40. The system of any of clauses 34 to 39, wherein the second wearable device comprises a patch configured to be carried on a torso of the subject.
41. The system of any of clauses 34 to 40, wherein the first wearable device comprises at least one of the one or more processors.
42. The system of any of clauses 34 to 40, wherein at least one of the one or more processors is in wireless communication with the first wearable device.
43. The system of any of clauses 34 to 42, wherein the first stimuli and the second stimuli are different types of stimuli.
44. The system of any of clauses 34 to 42, wherein the first stimuli and the second stimuli are the same type of stimuli.
45. A method for training one or more processors operably coupled to a first wearable device to detect defecation events of a subject, the method comprising:
   sensing, by a first defecation event sensor carried by the first wearable device, one or more first stimuli;
   sensing, by a second defecation event sensor carried by a second wearable device, one or more second stimuli;
   determining, by the one or more processors, occurrence of a detected defecation event of the subject based on the second stimuli; and
   associating, by the one or more processors, first stimuli sensed by the first defecation event sensor within a predetermined time period of the detected defecation event with defecation events of the subject.
46. The method of clause 45, further comprising training, by the one or more processors, a machine-learning algorithm for detecting defecation events of the subject with data indicative of first stimuli sensed by the first defecation event sensor that has been associated with defecation events of the subject.
47. The method of clause 45, wherein the first wearable device further comprises a wake-up sensor configured to sense one or more wake-up stimuli, the first wearable device being configured to transition from a sleep mode to an active mode in which the first wearable device is configured to sense defecation events of the subject via the first defecation event sensor when the wake-up sensor senses wake-up stimuli associated with defecation events of the subject, the method further comprising:
   associating, by the one or more processors, wake-up stimuli sensed by the wake-up sensor within a second predetermined time period of the detected defecation event with defecation events of the subject.
48. The method of any of clauses 45-47, wherein the one or more processors comprises a first processor operably coupled to the first defecation event sensor and a second processor operably coupled to the second defecation event sensor, wherein the first processor and the second processor are operably coupled to each other.
49. The method of any of clauses 45-48, wherein the one or more processors consists of a single processor operably coupled to the first defecation event sensor and the second defecation event sensor.
50. The method of any of clauses 45-48, wherein the first wearable device comprises a smartwatch.
51. The method of any of clauses 45-50, wherein the second wearable device comprises a patch configured to be carried on a torso of the subject.
52. The method of any of clauses 45-51, wherein the first wearable device comprises at least one of the one or more processors.
53. The method of any of clauses 45-51, wherein at least one of the one or more processors is in wireless communication with the first wearable device.
54. The method of any of clauses 45-52, wherein the first stimuli and the second stimuli are different types of stimuli.
55. The method of any of clauses 45-52, wherein the first stimuli and the second stimuli are the same type of stimuli.
56. A system for training one or more processors to detect defecation events of a subject, the system comprising:
   a wearable device, wherein the wearable device is configured to be carried on a body of the subject, the wearable device comprising a defecation event sensor configured to sense one or more stimuli;
   a mobile device configured to receive user input from the subject indicating a defecation time point at which a defecation event occurred; and
   one or more processors operably coupled with the defecation event sensor and the mobile device configured to associate stimuli sensed by the defecation event sensor within a predetermined time period of the defecation time point with defecation events of the subject.
57. The system of clause 56, wherein the one or more processors are further configured to train a machine-learning algorithm for detecting defecation events of the subject with data indicative of first stimuli sensed by the first defecation event sensor that has been associated with defecation events of the subject.
58. The system of clause 56-57, wherein:
   the wearable device further comprises a wake-up sensor configured to sense one or more wake-up stimuli;
   the wearable device is configured to transition from a sleep mode to an active mode in which the wearable device is configured to sense defecation events of the subject via the defecation event sensor when the wake-up sensor senses wake-up stimuli associated with defecation events of the subject; and
   the one or more processors are further configured to associate wake-up stimuli sensed by the wake-up sensor within a second predetermined time period of the detected defecation event with defecation events of the subject.
59. The system of any of clauses 56-58, wherein the mobile device comprises at least one of the one or more processors.
60. The system of any of clauses 56-59, wherein the wearable device comprises at least one of the one or more processors.
61. The system of any of clauses 56-60, wherein the wearable device comprises at least one of a smartwatch and a patch configured to be carried on a torso of the subject.
62. A method for training one or more processors operably coupled to a wearable device to detect defecation events of a subject, the method comprising:
   sensing, by a defecation event sensor carried by the wearable device, one or more stimuli;
   receiving user input via a mobile device of the subject indicating a defecation time point at which a defecation event occurred; and
   associating, by the one or more processors, stimuli sensed by the defecation event sensor within a predetermined time period of the defecation time point with defecation events of the subject.
63. The method of clause 62, further comprising training, by the one or more processors, a machine-learning algorithm for detecting defecation events of the subject with data indicative of first stimuli sensed by the first defecation event sensor that has been associated with defecation events of the subject.
64. The method of any of clauses 62-63, wherein the wearable device further comprises a wake-up sensor configured to sense one or more wake-up stimuli, the wearable device being configured to transition from a sleep mode to an active mode when the wake-up sensor senses wake-up stimuli associated with defecation events of the subject, the method further comprising:
   associating, by the one or more processors, wake-up stimuli sensed by the wake-up sensor within a second predetermined time period of the detected defecation event with defecation events of the subject.
65. The method of any of clauses 62-64, wherein the mobile device comprises at least one of the one or more processors.
66. The method of any of clauses 62-65, wherein the wearable device comprises at least one of the one or more processors.
67. The method of any of clauses 62-66, wherein the wearable device comprises at least one of a smartwatch and a patch configured to be carried on a torso of the subject.

## Claims

1. A system for sensing defecation events of a subject, the system comprising:
a wearable device configured to be carried on a torso of the subject and under lower-body clothing worn by the subject, the wearable device comprising
an optical sensor configured to sense increased light when the subject removes the lower-body clothing; and
a processor operably coupled to the optical sensor, the processor configured to determine occurrence of defecation events based at least in part on the increased light sensed by the optical sensor.

2. The system of claim 1, wherein the wearable device is operable in an active mode and a sleep mode, and the processor is configured to switch the wearable device from the sleep mode to the active mode upon determining removal of the lower-body clothing by the subject in response to the increased light sensed by the optical sensor.

3. The system of claim 2, wherein the optical sensor is a first sensor configured to sense light as a first stimulus, wherein the wearable device further comprises a second sensor configured to sense a second stimulus when the wearable device is in the active mode, the second stimulus being different than the first stimulus, and wherein the processor is operably coupled to the second sensor, the processor being configured to determine occurrence of defecation events of the subject based on signals received from the second sensor.

4. The system of claim 2, wherein the optical sensor is a first sensor configured to sense light as a first stimulus, wherein the wearable device further comprises a second sensor configured to sense a second stimulus when the wearable device is in the active mode, the second stimulus being different than the first stimulus, and wherein the processor is operably coupled to the second sensor, the processor being configured to determine occurrence of defecation events of the subject based on signals received from the first sensor and the second sensor.

5. The system of any of claims 3-4, wherein the second sensor is a mechanomyogram sensor.

6. The system of any of claims 1-5, wherein the wearable device further comprises a patch configured to be carried on the torso of the subject, the patch carrying the optical sensor and the processor.

7. The system of any of claims 1-5, wherein the wearable device further comprises a belt configured to extend around the torso of the subject, the belt carrying the optical sensor and the processor.
